Europäisches Patentamt

European Patent Office

Office européen des brevets

(11) Publication number: **0 210 055**
**A1**

(12) **EUROPEAN PATENT APPLICATION**

(21) Application number: 86305524.0

(22) Date of filing: 17.07.86

(51) Int. Cl.⁴: **C 07 C 67/08**
**B 01 D 13/04, C 07 C 69/14**

(30) Priority: 23.07.85 GB 8518575

(43) Date of publication of application:
28.01.87 Bulletin 87/5

(84) Designated Contracting States:
BE DE FR GB NL

(71) Applicant: BP Chemicals Limited
Belgrave House 76 Buckingham Palace Road
London, SW1W 0SU(GB)

(72) Inventor: Pearce, Graeme Keith
BP Chemicals Limited Salt End
Hedon Hull, HU12 8DS(GB)

(74) Representative: Fawcett, Richard Fennelly et al,
BP INTERNATIONAL LIMITED Patents Division Chertsey
Road
Sunbury-on-Thames Middlesex, TW16 7LN(GB)

(54) Esterification process.

(57) This invention relates to a process for producing liquid esters, especially liquid aliphatic esters, by carrying out the reaction in a reactor provided with a pervaporation membrane. The membrane enables continual removal of one or more reaction products from the reaction and thereby significantly reduces the energy input to the reaction for shifting the reaction equilibrium towards the desired ester product.

1

## ESTERIFICATION PROCESS

The present invention relates to an improved process for producing liquid esters by carrying out the esterification reaction in a reactor provided with a pervaporation membrane.

Pervaporation membranes are permeable membranes which when contacted on the upstream side thereof with a liquid enables the permeate emerging therefrom to be removed as vapour.

In esterification reactions, the reactants, products and byproducts are separated by conventional techniques. Moreover, such conventional methods such as distillation and separation have been used hitherto to drive the reaction towards high levels of ester production. For instance, in the case of producing ethyl acetate, the conventional method uses 2.5 te steam/te ethyl acetate product and most of this energy is required to remove water formed in the reaction.

It is an object of the present invention to use a pervaporation membrane to facilitate continuous removal of one or more reaction products from an esterification reaction thereby significantly reducing the energy input to the reaction for shifting the reaction equilibrium towards the desired ester product.

Accordingly, the present invention is a process for producing liquid esters by reaction of an alcohol with a carboxylic acid in the presence of an acidic catalyst characterised in that a pervaporation membrane is interposed in the process train comprising the reaction and product separation stages such that water of the reaction is continuously removed in situ from the reaction mixture

0210055

through the membrane.

The esterification reaction is performed with reactant carboxylic acid and alcohol which give rise to liquid esters. The ester is preferably an aliphatic ester. Specific examples of such esters include the acetates of methanol and ethanol.

Pervaporation membranes having a low acid resistance, i.e. capable of operating in a system in which the free organic acid level is no more than 15 mole% of the total reaction mixture, are preferred. For pervaporation membranes having a low acid resistance, the reaction is suitably carried out using a molar excess of the alcohol reactant with respect to the carboxylic acid reactant. In the case of ethyl acetate the molar ratio of ethanol to acetic acid is suitably 2:1 although with membranes of high acid resistance, molar ratios of 1:1 can be used.

The acidic catalyst may be heterogeneous or homogeneous. Where the acidic catalyst is heterogeneous, it is suitably an ion-exchange resin such as the Amberlyst (Registered Trade Mark) grades, e.g. Amberlyst A15. Where the acidic catalyst used is homogeneous it is suitably methane sulphonic acid or a toluene sulphonic acid such as para-toluene sulphonic acid.

The pervaporation membrane may be any of the commercialy available varieties provided that they have adequate resistance to an acidic environment of the type encountered in such esterification reactions. That is, the membrane should be able to maintain substantially its pervaporation characteristics under the reaction conditions. Examples of such membranes include membranes manufactured by GFT Ingenieur Buro fur Industrie Anlagen Plannung, which consist of a polyvinyl alcohol active layer cast onto polyacrylonitrile and is then in turn cast onto a non-woven polyester support, and Nafion (Registered Trade Mark) membranes, which are perfluorosulphonic acid cation exchange polymers and which may or may not be reinforced. Such membranes are stable in alcohols, esters and ketones and in low concentrations of carboxylic acids. These membranes also maintain their stability at working temperatures up to 100°C. However, other membranes having stability at higher temperatures e.g. up to 150°C can also be used.

The membranes allow a substantially aqueous permeate to be obtained in preference to the acid, alcohol and ester components in the reaction products. The removal of water shifts the equilibrium further in favour of the ester product.

A module of the pervaporation membrane may be interposed in the process train after the catalyst bed in the reactor. Alternatively a so-called sequential membrane reactor can be used, comprising a series of alternating reactors and membrane modules. The level of ester is sucessively increased after passage through each reactor, followed by dehydration in the membrane module.

Alternatively, commercial membranes or modules produced in the form of cylindrical concentric tubes for use in reverse osmosis may be chemicaly modified on the surface to function as the pervaporation membrane. An example of such reverse osmosis modules is the type produced by Paterson Candy International. In this case the membrane forms the inner cylindrical tube. These are the so-called integral membrane reactors. For the esterification reaction the core of the inner tube is packed with the solid heterogeneous catalyst, and the reactant acid and alcohol fed to the bottom and allowed to pass up the tube through the catalyst. Water generated during the esterification reaction is withdrawn continuously through the membrane along the length of the tubular module and the product ester (along with, in some cases, small amounts of unreacted acid and alcohol) collected at the top as the retentate emerging from the catalyst bed.

In the case of using integral membrane reactors, a series of such reactors could be used to allow complete and continuous removal of water from products. It would be possible to adjust the flow rate of the reactants through such a catalyst bed(s) in such a manner that with complete removal of water, the emergent product is substantially pure ethyl acetate.

It is desirable to carry out the esterification reaction at temperatures from 20°C to 150°C, preferably from 50°C to 120°C. The feed and/or membrane module have to be heated accordingly.

The esterification reaction can be carried out at any convenient pressure at which the reaction mixture is liquid at the reaction temperature, e.g. 1 bar absolute to 10 bar absolute, preferably 1 bar absolute to 4 bar absolute.

The present invention is further illustrated with reference to the following Examples.

In these Examples, the process has been described with reference to the production of ethyl acetate.

## Examples

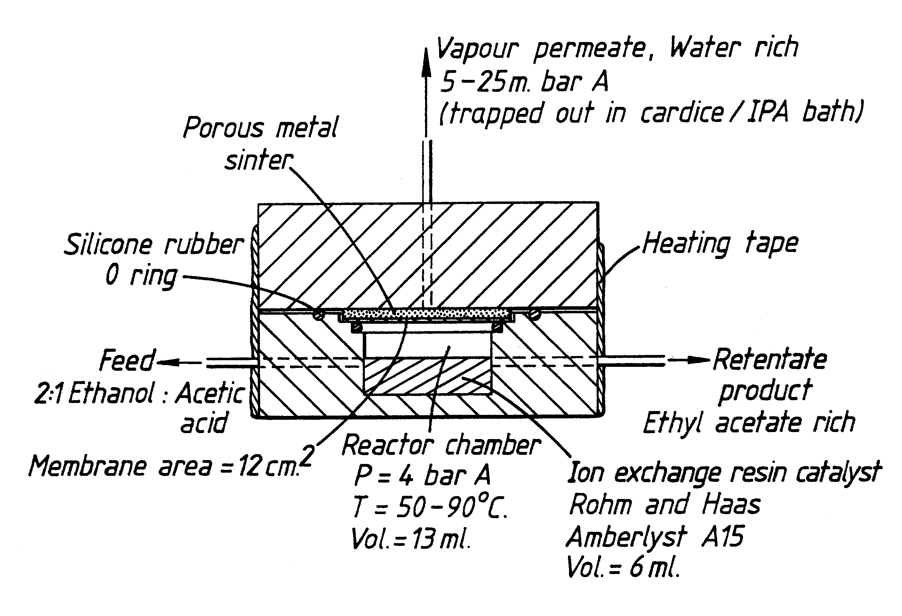

A sketch of the pervaporation test cell summarising the experimental conditions is shown in Figure 1. Feeds containing a 2:1 molar ratio of ethanol to acetic acid were pumped through the heated membrane cell at a measured flow rate. The cell chamber contained an ion exchange resin catalyst, Amerlyst A15 (Rohm and Haas), which catalysed the esterification reaction. Two types of experiment were carried out.

## Blank Runs

These runs enabled the catalytic activity of the ion exchange resin to be measured for different conditions.

## Pervaporation Runs

Withdrawal of a water rich vapour permeate from the chamber through the pervaporation membrane enhanced the production of ethyl acetate. The pervaporation runs were carried out at comparable flow and temperature conditions to the blank runs giving a direct measure of the improvement resulting from the use of pervaporation. Feed, retentate and permeate samples were analysed at frequent intervals by Gas Chromatography.

Three types of commercialy available pervaporation membrane were used:

GFT Membrane

Nafion 117

Nafion 324

Details of the membranes are given below.

## GFT Membrane

This membrane consists of a polyvinyl alcohol active layer cast onto polyacrylonitrile, which in turn is cast onto a non-woven terylene support.

The membrane is stable in alcohols, esters, ketones and low concentrations of carboxylic acids. Working temperatures up to 100°C can be used.

## Nafion Membrane

Nafion membrane is a perfluorosulphonic acid cation exchange polymer. Nafion 117 is an unreinforced film used for $H_2O$ and HCl electrolysis. Nafion 324 is a reinforced copolymer composite membrane used for NaOH production.

The membranes have very high chemical stability. Considerable expansion occurs with increasing temperature (which led to partial failure of Nafion 117 at 90°C). The cost of the membrane is approximately £200 to £300/m$^2$.

## Results

Ethanol rich feeds were used containing a 2:1 molar ratio of ethanol:acetic acid.

Table 1 lists the initial feed composition, the equilibrium composition, and the equilibrium composition with total water removal. As the mixture moves towards equilibrium the ethyl acetate level approaches a maximum level of 48% w/w; if the water is removed by pervaporation this increases to 66% w/w. The actual ethyl acetate levels achieved at 70°C and 90°C for the blank runs and for the three membrane pervaporation runs can be seen in Tables 2 and 3. The difference in ethyl acetate levels between the membrane runs and the blank run is the enhanced ethyl acetate production. Tables 4, 5 and 6 show the variation of enhanced ethyl acetate production with flow rate at different temperatures for each of the three membranes. The membrane performance is shown in Table 7.

## Discussion

### Catalytic Activity of Amberlyst Al5

An assessment of the catalytic activity of Amberlyst Al5 can be made by consideration of the blank run results in Tables 2 and 3. A low ethyl acetate production rate is observed at high flow rates due to the low cell volume; as the flow rate is reduced, the ethyl acetate level begins to rise sharply toward the equilibrium value of 48% w/w. The Liquid Hourly Space Velocity, LHSV, which relates the flow rate to the catalyst bed volume, can be used as a measure of the effectiveness of a heterogeneous catalyst system. Table 3 shows that at 90°C, the catalyst demonstrates an encouraging level of activity with an LHSV in the range 2 to 5.

Enhancement of Ethyl Acetate Production by Pervaporation

Tables 2 and 3 show that at high flow rates the enhancement of ethyl acetate production is modest since the membrane flux and membrane area are low in comparison with the residence time of the liquid in the cell. However, as the flow rate is reduced, it is evident that substantial enhancements of ethyl acetate are possible, particularly at 90°C, as the reactor contents become dehydrated to a significant degree. The enhancements derived from these Examples are shown for each of the three membranes in Tables 4, 5 and 6. Despite the low water flux of the GFT membrane, the enhancement in ethyl acetate production was nearly comparable to the Nafion membrane.

Table 1

Feed and Limiting Product Compositions

|  | Feed | | Equilibrium Composition | | Equilibrium Composition with total water removal | |
|---|---|---|---|---|---|---|
|  | % mole | % w/w | % mole | % w/w | % mole | % w/w |
| Acetic acid | 33.3 | 39.5 | 5.5 | 6.5 | 0 | 0 |
| Ethanol | 66.7 | 60.5 | 38.8 | 35.3 | 50 | 34.3 |
| Ethyl acetate | 0 | 0 | 27.8 | 48.3 | 50 | 65.7 |
| Water | 0 | 0 | 27.8 | 9.9 | 0 | 0 |

### Table 2
### Ethyl Acetate Composition and Flowrate for 70°C Run Temperature

| Blank | | GFT | | Nafion 324 | | Nafion 117 | |
|---|---|---|---|---|---|---|---|
| EA % w/w | Flow ml/hr | EA % w/w | Flow ml/hr | EA % w/w | Flow ml/hr | EA % w/w | Flow ml/hr |
| 12.4 | 42 | 11.3 | 67 | 11.5 | 64 | 17.0 | 46 |
| 10.9 | 58 | 11.5 | 69 | 10.6 | 70 | 12.9 | 67 |
| 10.8 | 64 | 5.7 | 160 | 6.1 | 139 | 6.0 | 160 |
| 5.2 | 130 | 5.4 | 162 | 6.5 | 142 | 6.0 | 190 |
| | | 5.2 | 167 | | | | |

### Table 3
### Ethyl Acetate Composition and Flowrate for 90°C Run Temperature

| Blank | | GFT | | Nafion 324 | | Nafion 117 | |
|---|---|---|---|---|---|---|---|
| EA % w/w | Flow ml/hr | EA % w/w | Flow ml/hr | EA % w/w | Flow ml/hr | EA % w/w | Flow ml/hr |
| 21.4 | 38 | 25.0 | 57 | 25.0 | 69 | 21.8 | 53 |
| 9.9 | 160 | 23.2 | 62 | 23.2 | 72 | 21.2 | 54 |
| | | 19.6 | 75 | 12.1 | 140 | 10.0 | 167 |
| | | 12.4 | 155 | 10.8 | 165 | 10.6 | 178 |
| | | 12.1 | 159 | | | | |

### Table 4
### Enhanced Ethyl Acetate Production vs Flowrate for GFT Membrane

| 50°C | | 70°C | | 90°C | |
|---|---|---|---|---|---|
| Increase in EA % w/w | Flow ml/hr | Increase in EA % w/w | Flow ml/hr | Increase in EA % w/w | Flow ml/hr |
| 2.2 | 40 | 2.4 | 40 | 6.2 | 60 |
| 1.6 | 80 | 1.8 | 80 | 4.1 | 80 |
| 0.75 | 160 | 1.3 | 160 | 2.2 | 160 |

## Table 5
### Enhanced Ethyl Acetate Production vs Flowrate for Nafion 117

| 70°C | | 90°C | |
|---|---|---|---|
| Increase in EA % w/w | Flow ml/hr | Increase in EA % w/w | Flow ml/hr |
| 4.6 | 40 | 5.8 | 40 |
| 1.7 | 80 | 3.1 | 80 |
| 1.0 | 160 | 2.2 | 160 |

## Table 6
### Enhanced Ethyl Acetate Production vs Flowrate for Nafion 324

| 50°C | | 70°C | | 90°C | |
|---|---|---|---|---|---|
| Increase in EA % w/w | Flow ml/hr | Increase in EA % w/w | Flow ml/hr | Increase in EA % w/w | Flow ml/hr |
| 1.1 | 40 | 2.0 | 40 | 8.2 | 70 |
| 0.7 | 80 | 1.4 | 80 | 5.3 | 80 |
| 0.25 | 160 | 1.2 | 160 | 1.1 | 160 |

## Table 7
### Membrane Performance for 98% w/w Organic Feed at 70°C

| Membrane | Water Flux kg/M$^2$/day | Permeate Composition % w/w water |
|---|---|---|
| GFT | 1.4 | 65 |
| Nafion 117 | 5.8 | 15 |
| Nafion 324 | 16.0 | 6 |

Claims:

1. A process for producing liquid esters by reaction of an alcohol with a carboxylic acid in the presence of an acidic catalyst characterised in that a pervaporation membrane is interposed in the process train comprising the reaction and product separation stages such that water of the reaction is continuously removed in situ from the reaction mixture through the membrane.

2. A process according to claim 1 wherein the ester product is an aliphatic ester.

3. A process according to claim 1 or 2 wherein the reaction has a free acid level below 15 mole% of the total reaction mixture.

4. A process according to any one of the preceding claims wherein the reaction is carried out using a molar excess of the alcohol reactant with respect to the carboxylic acid.

5. A process according to any one of the preceding claims wherein the membranes maintain their physical characteristics and stability up to 150°C.

6. A process according to any one of the preceding claims wherein the membrane is either a polyvinyl alcohol active layer cast on to polyacrylonitrile which is then cast on to a non-woven polyester support, or, a perfluorosulphonic acid cation exchange polymer.

7. A process according to any one of the preceding claims wherein the membrane is used (i) in the form of one or more modules interposed in the process train after the catalyst bed in the reactor, or (ii) as a sequential membrane reactor which comprises a series of alternating reactors and modules, or (iii) as an integral membrane reactor which is in the form of two concentric cylindrical modules in which the membrane forms the inner tube having a core of the catalyst.

8. A process according to any one of the preceding claims wherein the reaction is carried out at a temperature from 20-150°C.

9. A process according to any one of the preceding claims wherein the acidic catalyst is a heterogeneous acidic catalyst.

10. A process according to claim 9 wherein the heterogeneous acidic catalyst is an ion-exchange resin.

11. A process according to any one of the preceding claims as hereinbefore described with reference to the Examples.

Vapour permeate, Water rich
5−25m. bar A
(trapped out in cardice / IPA bath)

Porous metal sinter

Heating tape

Silicone rubber 0 ring

Feed
2:1 Ethanol : Acetic acid

Retentate product
Ethyl acetate rich

Membrane area = 12 cm.$^2$

Reactor chamber
$P = 4$ bar A
$T = 50−90°C.$
Vol. = 13 ml.

Ion exchange resin catalyst
Rohm and Haas
Amberlyst A15
Vol. = 6 ml.

1/1

0210055

European Patent Office

**EUROPEAN SEARCH REPORT**

Application number

| | DOCUMENTS CONSIDERED TO BE RELEVANT | | EP 86305524.0 |
|---|---|---|---|

| Category | Citation of document with indication, where appropriate, of relevant passages | Relevant to claim | CLASSIFICATION OF THE APPLICATION (Int Cl 4) |
|---|---|---|---|
| A | EP - A1 - 0 105 111 (CHEM. WERKE HULS) <br> * Examples; claims * <br> -- | 1,2,4, 8-11 | C 07 C 67/08 <br> B 01 D 13/04 <br> //C 07 C 69/14 |
| A | GB - A - 1 394 651 (BP CHEMICALS) <br> * Claims * <br> -- | 1,2,4, 8,9,11 | |
| A | EP - A2 - 0 096 339 (GFT) <br> * Examples; claims * <br> ---- | 1,5,6 | |

| | TECHNICAL FIELDS SEARCHED (Int. Cl.4) |
|---|---|
| | C 07 C 67/00 <br> C 07 C 69/00 <br> B 01 D |

The present search report has been drawn up for all claims

| Place of search | Date of completion of the search | Examiner |
|---|---|---|
| VIENNA | 27-10-1986 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82